# EUROPEAN PATENT APPLICATION

(11) **EP 0 969 003 A2**
(43) Date of publication of application: **05.01.2000**
(21) Application number: 99304580.6
(22) Date of filing: 11.06.1999
(51) Int. Cl.: C07D 221/10, C07C 43/192, C07C 43/313, C07C 323/21

(54) **Process for the preparation of benzo[f]quinolinone derivatives and intermediates**

(30) Priority: 18.06.1998 US 89749 P
(71) Applicant: ELI LILLY AND COMPANY, Indianapolis, Indiana 46285 (US)
(72) Inventor: Heath, Perry Clark, Indianapolis, Indiana 46217 (US)
(74) Representative: Vaughan, Jennifer Ann

(57) **Abstract**

The present invention provides a novel process for preparing benzo(f)quinolinones of the formula I 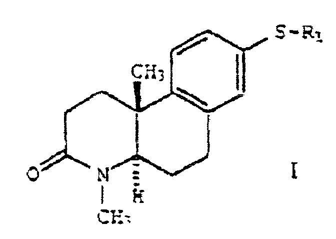 wherein R¹ represents:
2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzoisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted; and the intermediates of the formulae A,B,C and D.

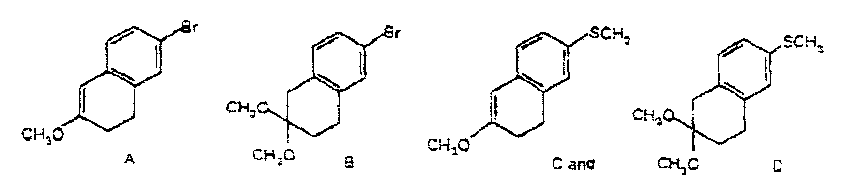

## Description

The present invention belongs to the fields of organic chemistry, pharmaceutical chemistry and chemical manufacture, and provides a convenient and economical process for preparing benzo[f]quinolinones which are useful as 5α-reductase inhibitors and provides intermediate compounds for the preparation of such pharmaceuticals.

A currently active field of pharmaceutical research is the inhibition of 5α-reductase, the enzyme which converts testosterone to dihydro-testosterone, a more potent androgen. It has been demonstrated that inhibitors of 5α-reductase can block the formation of dihydrotestosterone and ameliorate a number of highly undesirable conditions, including male pattern baldness and benign prostatic hypertrophy. Audia *et al.,* have disclosed a series of benzo[f]quinolinone compounds which are 5α-reductase inhibitors. *See:* U.S. Patents 5,239,075 and 5,541,190; *Tet. Let.,* 44, 7001 (1993); *J. Med. Chem., 36*, 421 (1993); and European Patent Publication 0703221.

The present invention provides a novel process for preparing benzo[f]quinolinones which are effective inhibitors of 5α-reductase. The present process is more efficient than prior processes and is amenable to large-scale synthesis. This invention also provides intermediate compounds for the preparation of such pharmaceuticals.

The present invention provides a novel process for preparing benzo[f]quinolinones and provides intermediates useful in preparing benzo[f]quinolinones. More specifically, the present invention is directed to a process for preparing a compound of the formula I 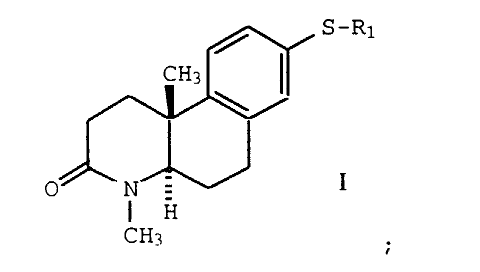 wherein R¹ represents:
2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzoisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted with 1-3 functionalities chosen from the group consisting of trifluoromethyl, trifluoroethoxy, C₁-C₄ alkyl, trifluoromethoxy, hydroxy, C₁-C₃ alkoxy, nitro, C₁-C₃ alkylthio, C₁-C₆ alkanoyl, phenyl, oxo, phenoxy, phenylthio, C₁-C₃ alkylsulfinyl, C₁-C₃ alkylsulfonyl, cyano, amino, C₁-C₃ alkylamino, diphenylmethylamino, triphenylmethylamino, benzyloxy, benzylthio, (mono-halo, nitro or CF₃)benzyl(oxy or thio), di(C₁-C₃ alkyl, C₃-C₆ cycloalkyl, or C₄-C₈ cycloalkylalkyl)amino, (mono-C₁-C₃ alkyl, C₁-C₃ alkoxy or halo)(phenyl, phenoxy, phenylthio, phenylsulfonyl or phenoxysulfonyl), C₂-C₆ alkanoylamino, benzoylamino, diphenylmethylamino(C₁-C₃ alkyl), aminocarbonyl, C₁-C₃ alkylaminocarbonyl, di(C₁-C₃ alkyl)aminocarbonyl, halo-C₁-C₆ alkanoyl, aminosulfonyl, C₁-C₃ alkylaminosulfonyl, di(C₁-C₃ alkyl)aminosulfonyl, phenyl(oxy or thio)(C₁-C₃ alkyl), (halo, C₁-C₃ alkyl or C₁-C₃ alkoxy)phenyl(oxy or thio)(C₁-C₃ alkyl), benzoyl, or (amino, C₁-C₃ alkylamino or di(C₁-C₃ alkyl)amino)(C₁-C₃ alkyl).

An aspect of the invention comprises: converting a ketone of the formula 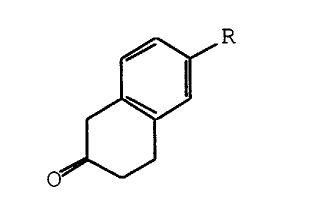 wherein R is halogen, preferably bromo;
to a protected ether, preferably employing trimethyl orthoformate in methanol in the presence of an acid catalyst;
reacting the protected ether with a reactive alkyllithium compound, such as, n-butyllithium and a sulfur transfer reagent, such as, dimethyl disulfide, to afford an *S*-methylated ether compound; and
deprotecting the *S*-methylated ether compound to afford a methylthiotetralone compound of the formula II

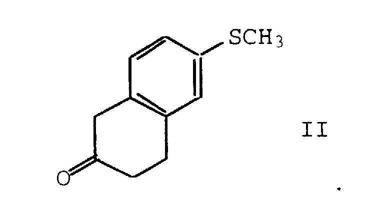

According to another aspect, the invention comprises converting the compound of formula II to a compound of the formula I. One such preferred process comprises reacting the compound of formula II with (*R*)-(+)- phenethylamine to afford a compound of the formula III 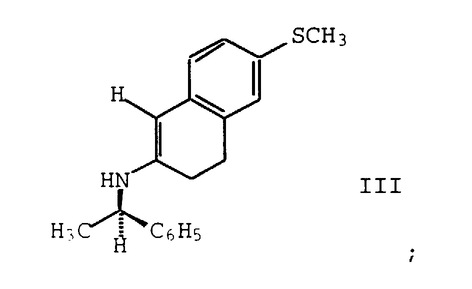 reacting the compound of formula III with a strong lithium base to afford a lithioenamine compound of the formula IV 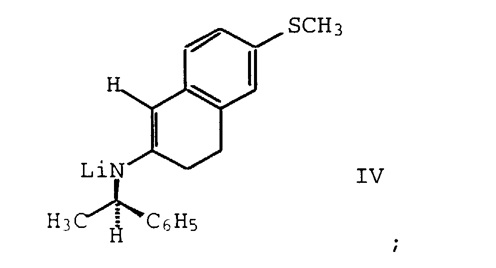 methylating the resulting lithioenamine of the formula IV to a compound of the formula V, for example, by reacting the resulting lithioenamine with methyl iodide in an ether solvent to prepare the compound of formula V 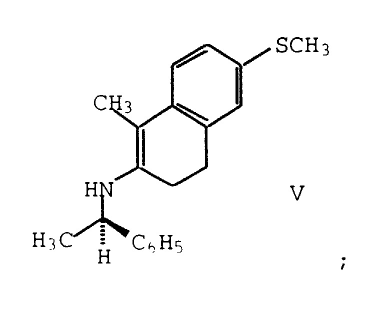 reacting the compound of formula V with an acyl halide or an anhydride of acrylic acid to prepare a compound of the formula VI 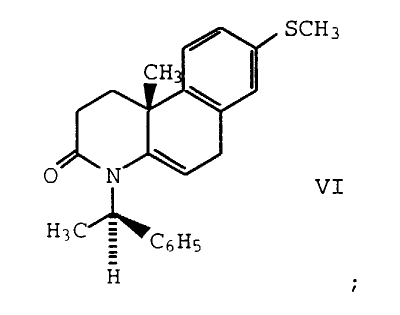 quenching the reaction with base, and combining the residue comprising the compound of formula VI with an appropriate silane and trifluoroacetic acid in the absence of a solvent to prepare a compound of the formula VII 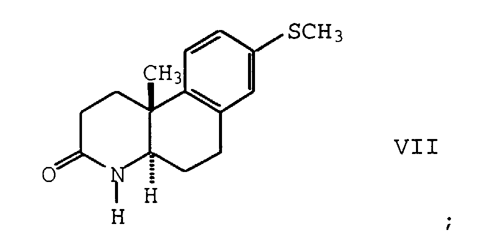 reacting the compound of formula VII with a methyl halide, for example, methyl iodide in a reaction mixture comprising an organic solvent and a strong base to afford an arylmethylsulfide compound of the formula VIII 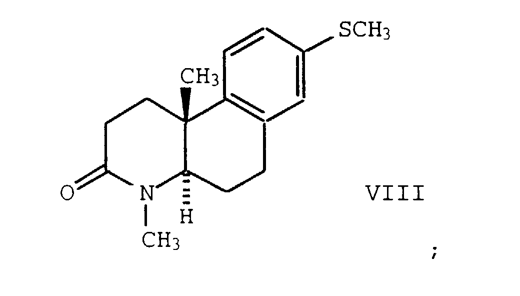 oxidizing the compound of formula VIII to a sulfoxide compound of the formula IX 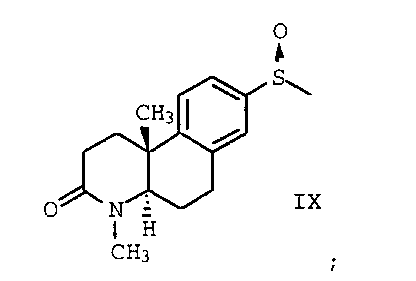 reacting the sulfoxide compound of the formula IX with an acylating agent to afford a Pummerer rearrangement product; reacting the Pummerer rearrangement product with an electrophile selected from the group consisting of A-R¹ wherein A is a leaving group and R¹ represents: 2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted with 1-3 groups chosen from the group consisting of trifluoromethyl, trifluoroethoxy, C₁-C₄ alkyl, trifluoromethoxy, hydroxy, C₁-C₃ alkoxy, nitro, C₁-C₃ alkylthio, C₁-C₆ alkanoyl, phenyl, oxo, phenoxy, phenylthio, C₁-C₃ alkylsulfinyl, C₁-C₃ alkylsulfonyl, cyano, amino, C₁-C₃ alkylamino, diphenylmethylamino, triphenylmethylamino, benzyloxy, benzylthio, (mono-halo, nitro or CF₃)benzyl(oxy or thio), dil(C₁-C₃ alkyl, C₃-C₆ cycloalkyl, or C₄-C₈ cycloalkylalkyl)amino, (mono-C₁-C₃ alkyl, C₁-C₃ alkoxy or halo)(phenyl, phenoxy, phenylthio, phenylsulfonyl or phenoxysulfonyl), C₂-C₆ alkanoylamino, benzoylamino, diphenylmethylamino(C₁-C₃ alkyl), aminocarbonyl, C₁-C₃ alkylaminocarbonyl, di(C₁-C₃ alkyl)aminocarbonyl, halo-C₁-C₆ alkanoyl, aminosulfonyl, C₁-C₃ alkylaminosulfonyl, di(C₁-C₃ alkyl)aminosulfonyl, phenyl(oxy or thio)(C₁-C₃ alkyl), (halo, C₁-C₃ alkyl or C₁-C₃ alkoxy)phenyl(oxy or thio)(C₁-C₃ alkyl), benzoyl, or (amino, C₁-C₃ alkylamino or di(C₁-C₃ alkyl)amino)(C₁-C₃ alkyl); in the presence of a phase transfer catalyst, ahydride reducing reagent and a base, to prepare a compound of formula I.

The preferred intermediates of the present invention have the formulas :

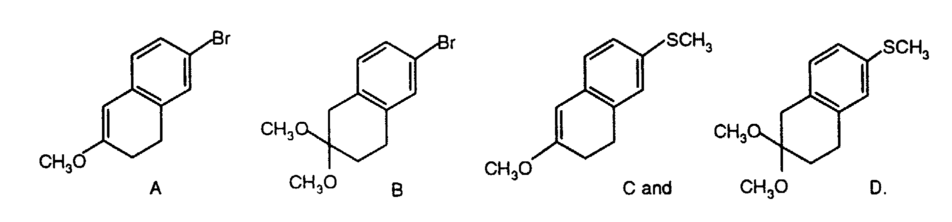

Starting materials for the compounds in the claimed process are either commercially available, known in the art, or can be prepared by methods known in the art, for example, see. Audia *et al.* in U.S. Patent 5,239,075, issued August 24, 1993 and European Patent Publication 0703221.

Throughout this document, all temperatures will be described in degrees Celsius and all expressions of concentration, percentage and proportion will be expressed in weight units, except for mixtures of solvents, which will be described in volume units, unless otherwise stated.

References to compounds in this document include the pharmaceutically acceptable salts of such compounds, unless otherwise stated.

The various positions on the benzo[f]quinoline ring are indicated below.

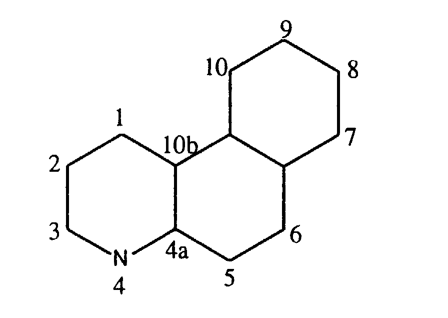

The spatial configuration of the group at 10b and the hydrogen atom at 4a are required. The reader will understand that many of the compounds can exist in two or more stereochemical forms, and that all stereochemical forms are included in the present invention. In some of the compounds prepared or described below, single enantiomers are prepared in pure form and are identified by (+) or (-) nomenclature. In other cases, the mixture of diastereomers is prepared.

The group S-R¹ occupies the 8 position.

The term "halogen" and "halo" includes chloro, bromo, fluoro and iodo.

The various alkyl groups, such as C₁-C₄ alkyl and the like include groups such as methyl, ethyl, propyl, isopropyl, *t*-butyl, *n*-butyl and isobutyl. Alkenyl and alkynyl groups constitute linking groups which are bivalent and are bonded to two other groups. For example, C₂-C₄ alkenyl includes ethenyl, 2-propenyl, 3-butenyl and 2-butenyl; and C₂-C₄ alkynyl includes, for example, ethynyl, 2-propynyl, 2-butynyl and iso-2-butynyl.

The group C₁-C₆ alkanoyl includes such groups as formyl, acetyl, propionyl, isobutyryl, 2-ethylpropionyl and hexanoyl. The group C₃-C₆ cycloalkyl includes cyclopropyl, cyclobutyl, cyclopentyl and cyclohexyl, and the group C₄-C₈ cycloalkylalkyl includes, for example, cyclopropylmethyl, cyclohexylethyl, cyclobutylbutyl and cyclohexylmethyl.

Terms such as halo-C₁-C₆ alkanoyl, halophenyl or C₁-C₃ alkylphenyl refer to the indicated basic group having substituted on it 1, 2, or 3 halo or C₁-C₃ alkyl groups as may be described in the individual case.

The present process prepares compounds of formula I all having the benzo[f]quinoline nucleus, on the benzo ring of which is substituted a cyclic group R¹ linked to the benzoquinoline through a sulfur linker. The R¹ groups may be substituted with additional organic groups, and may bear as many as three of the indicated substituent groups. Multiple substituents may all be the same or may be different.

Certain aspects of the process are preferred and will be mentioned below specifically. It will be understood that the following aspects are each important individually, and also that preferred aspects may be combined to create further, more limited or more expansive, preferred aspects.

### Synthesis of 6-Methylthio-2-tetralone

The starting material for the synthesis of 6-methylthio-2-tetralone is a 4-halophenylacetic acid (4-bromophenylacetic acid will be used for an example). 4-Bromophenyacetic acid is commercially available (Aldrich Catalog Handbook of Fine Chemicals 1994-5, page 233) or may be prepared by procedures well-known to those skilled in the art. 4-Bromophenylacetic acid is employed to prepare 6-bromo-2-tetralone.

6-Bromo-2-tetralone is prepared by reacting 4-bromophenylacetic acid with thionyl chloride, phosphorus trichloride, oxalyl chloride, or phosphorus pentachloride, under conditions known to those skilled in the art, to afford 4-bromophenacetyl chloride (Scheme 1). Preferably thionyl chloride and methylene chloride are employed to afford the 4-bromophenacetyl chloride. By a Friedel-Crafts acylation reaction of 4-bromophenacetyl chloride with ethylene gas in the presence of a Lewis acid catalyst and an inert or substantially inert solvent or mixture of solvents, ring closure is effected to afford the 6-bromo-2-tetralone.

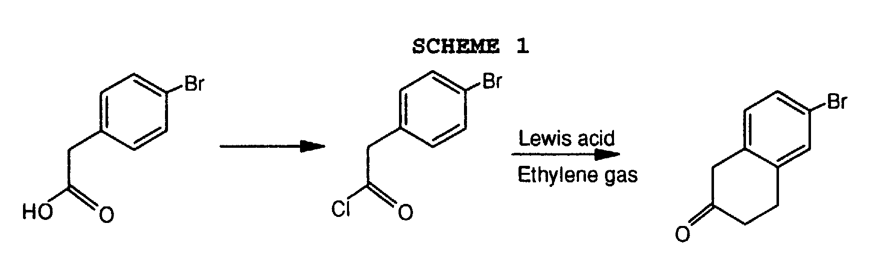

Suitable Lewis acid catalysts include AlBr₃, AlCl₃, AlI₃, GaCl₃, FeCl₃, SbCl₅, ZrCl₄, SnCl₄, BCl₃, BF₃, SbCl₃ and the like, preferably, AlCl₃. Solvents used for this reaction include carbon disulfide, methylene chloride, nitromethane, 1,2-dichloroethane, nitrobenzene, and the like, preferably methylene chloride.

The ketone group of 6-bromo-2-tetralone is protected by reaction with trimethyl orthoformate in a solvent, such as, methanol, to afford the corresponding ethers according to the following Scheme 2. Hydrochloric acid, formed in situ by addition of thionyl chloride, acts as a catalyst in this reaction. One skilled in the art would recognize that other acid catalysts may be employed, e.g. p-toluenesulfonic acid, phosphoric acid, etc. Temperatures from about 20°C to about 65°C are employed using standard cooling procedures.

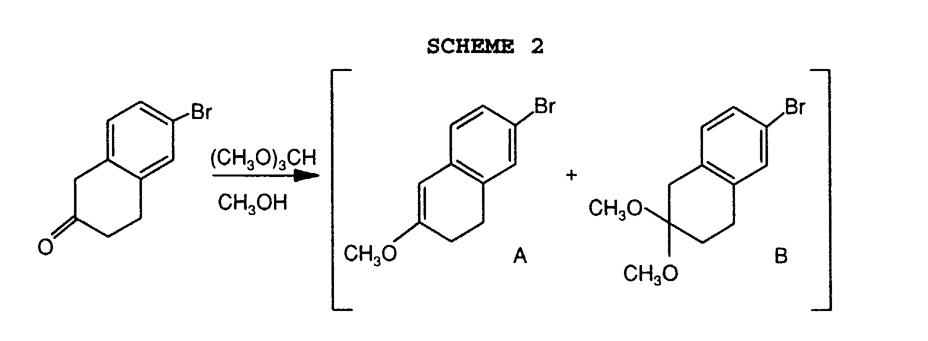

Halogen-metal exchange is then afforded by reacting the 6-bromo protected ethers (A and B) with a reactive organolithium compound, preferably *n*-butyllithium. Alkylation of the resulting lithium species with an appropriate sulfur transfer reagent, for example, a sulfenyl halide or dimethyl disulfide affords the ether protected 6-methylthio-2-tetralones. Deprotection is afforded by methods known to those skilled in the art. A preferred method of deprotection is treatment with aqueous acid, preferably hydrochloric acid, to afford the desired 6-methylthio-2-tetralone compound (Schemes 3 and 4).

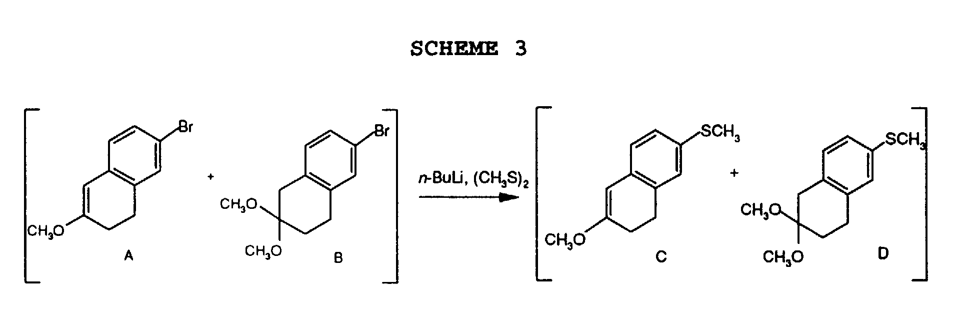

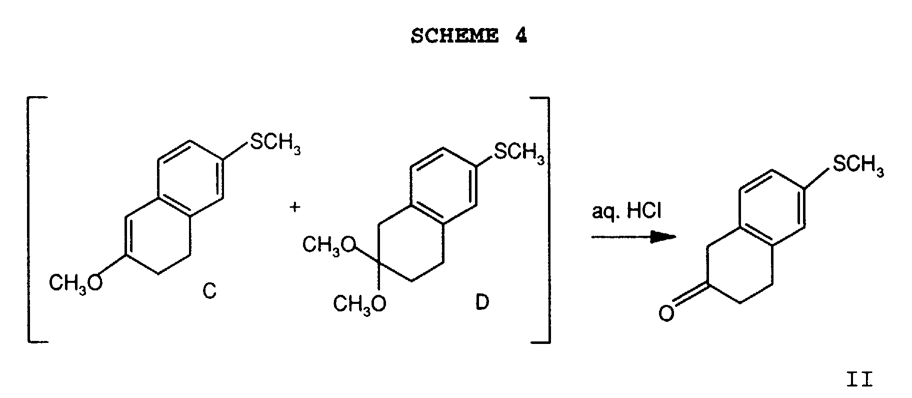

All transformations, starting from the 4-bromophenylacetic acid to the desired 6-methylthio-2-tetralone can be carried out conveniently in a single reaction flask without isolation of the intermediates. This improved process offers improved yields, a one-pot reaction, uses readily available reactants and is suitable for large-scale production. The desired 6-methylthio-2-tetralone may be isolated by standard methods, preferably by crystallization from the reaction mixture.

### Alkylation and Aza-annulation

A previous synthesis for the intermediate compound of the formula VII 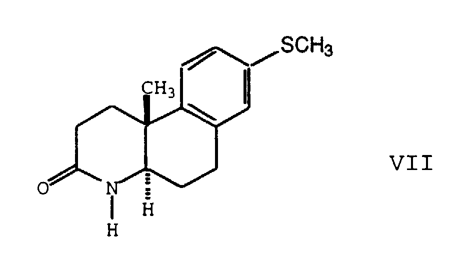 was taught in U.S. Patent 5,239,075 and U.S. Patent Application Serial No. 08/443,994, each of which is incorporated by reference herein. Another synthesis of the compound of formula VII, of which this invention constitutes an improvement, was shown in European Patent Publication 0564193. The methylthio group of the compound of formula VII is located at the 8-position. This preferred process of preparing the compound of formula VII, described below, can be carried out without purification or isolation of the intermediate products.

6-Thiomethyl-2-tetralone is reacted with (*R*)-(+)-phenethylamine to prepare the intermediate of the formula III

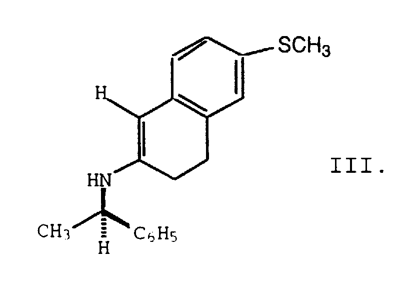

The reaction is conveniently carried out at elevated temperature, particularly reflux temperature, in toluene in the presence of a strong acid such as *p*-toluenesulfonic acid. Water must be removed as it is formed in this reaction, and the absence of water being formed is an indication of completion of the reaction. A slight excess of phenethylamine, such as about 1.05-1.10 equivalents, should be used. Alternatively, tetrahydrofuran may be used as the solvent, and it is particularly convenient in that case to use molecular sieves to dehydrate the reaction mixture, using at least twice the weight of molecular sieves compared to the amount of water which will be released by the process.

The above phenethylamino compound is lithiated with, for example, *n*-butyllithium or with lithium diisopropylamide to afford a compound of the formula IV

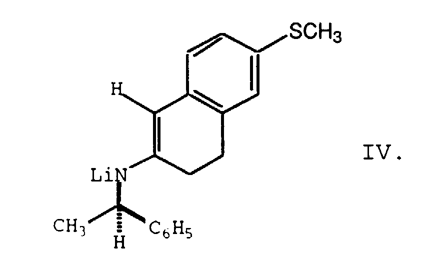

When the reaction is carried out, as is preferred, with lithium diisopropylamide, the best results are obtained if the lithium diisopropylamide is freshly generated from diisopropylamine and *n*-butyllithium immediately before use in the process. A substantial excess, about 15-25%, of lithium diisopropylamide should be used for best results.

The lithium diisopropylamide reaction is best carried out in tetrahydrofuran at a low temperature in the range of about -100° to about 0°, preferably about -78° to about -10°. The phenethylamino compound need not be purified or isolated, but the first reaction mixture should be evaporated under vacuum and the residue taken up in tetrahydrofuran. It is preferred to add the phenethylamino material, in solution, to a solution of lithium diisopropylamide in cold tetrahydrofuran; the opposite manner of addition is operable but provides lower yields. In general, the reaction may be carried out in less than one hour.

The lithio compound is difficult to isolate and purify, and so it should be introduced into the process of the present invention as a solution in the lithiation reaction mixture.

### Alkylation

The lithio compound is methylated, for example by reacting the resulting lithioenamine with methyl iodide to provide the compound of the formula V

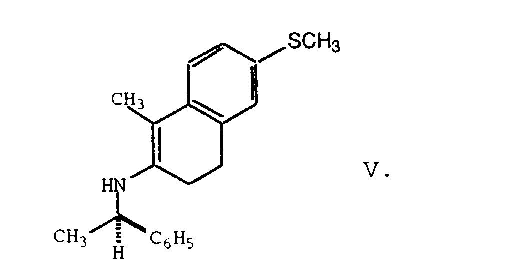

Dimethyl sulfate, methyl bromide, methyl chloride, methyl iodide, and the like, may be employed to methylate the lithioenamine. It is advisable to use about 15-25% of excess methyl iodide, and to carry out the process in a solvent, preferably an ether solvent, such as diethyl ether, methyl *t*-butyl ether or, preferably, tetrahydrofuran. The reaction is very rapid at low temperatures in the range of about -100° to about -50°, most preferably, about -80° to about -60°. Reaction times in the range of from about a few minutes to about one hour are adequate, and a 20 minute reaction time is preferred.

If the lithio compound is in the form of the reaction mixture from lithiation with lithium diisopropylamide, and the reaction mixture therefore contains the residual diisopropylamine, that amine must be neutralized before methylation. Most conveniently, the methyl iodide mixture is allowed to warm to a temperature close to 0°, and a sufficient amount of methanesulfonic acid is added to neutralize the diisopropylamine. Other strong acids may be used, but methanesulfonic acid is particularly convenient and preferred because the resulting methanesulfonate salt of diisopropyl-amine is only slightly soluble and therefore may be easily removed by simple filtration or centrifugation.

### Aza-Annulation Step

The reaction mixture comprising the compound of the formula V is combined with an acyl halide or an anhydride of acrylic acid or acryloyl chloride, or the like, to initiate the aza-annulation reaction which forms the compound of formula VI

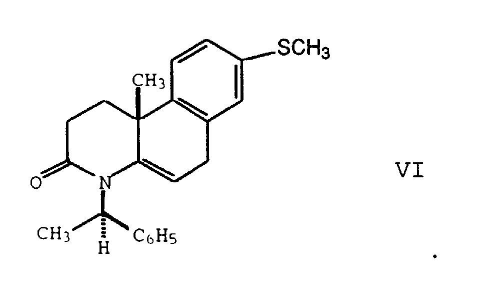

It is best to generate the acrylic anhydride, the preferred reagent, immediately before use by the reaction of acryloyl chloride and acrylic acid, using triethylamine and stabilizers, such as hydroquinone and butylated hydroxytoluene, in tetrahydrofuran.

The aza-annulation is best carried out by adding the acrylic anhydride or acryloyl chloride at a very low temperature, such as from about -100° to about -70°, and allowing the mixture to warm very slowly with stirring to a temperature in the range of about -20° to about 0°, or even up to about 10°-20°. A period of 12-15 hours is a reasonable period of time to allow the mixture to warm. When the reaction has gone as far toward completion as is desired, the reaction is quenched by addition of solid sodium bicarbonate. It is preferred to use from about 1.5 to about 4 equivalents of base, most preferably about 2 equivalents. The base may be added as a solution, for example, in water or in an aqueous solvent such as water/dimethylaminopyridine, but it is preferred to add the base in solid form. The reaction mixture is stirred with the quenching base for a brief period, and then the mixture is filtered, the volatiles are removed, and the solvent may be replaced with an ether solvent, preferably diethyl ether, and the organic solution may then be worked-up by washing with aqueous base and aqueous acid, and perhaps with additional purification steps such as a wash with a saturated salt solution. If such work-up steps are used, the solution is then dehydrated and evaporated under vacuum to obtain the non-volatile portions of the reaction mixture, containing the final intermediate of formula VI. On the other hand, the residue from the quenched reaction mixture may be carried on without work-up, if desired.

### Reduction-Cleavage Step

The residue from the aza-annulation step is cooled, and a chilled mixture of an appropriate silane and trifluoroacetic acid is added. An appropriate silane being a soluble silane, for example, a dialkylsilane or trialkylsilane or the like. The addition should take place at a low temperature in the range of from about -40° to about 0°, and no other solvent is used. A large quantity of trifluoroacetic acid, in the range of about 10-50 equivalents, most preferably about 20-30 equivalents is used. The preferred trialkylsilane is triethylsilane, although trimethylsilane, trisopropylsilane and the like may also be used. A substantial excess of trialkylsilane, in the range of about 5-20 equivalents, most preferably about 7-15 equivalents is used. The mixture is stirred for about 10-20 hours while it is allowed to warm slowly to about 30°, and then the mixture is slowly heated to an elevated temperature, preferably reflux temperature, and is stirred at that temperature for a few hours, such as about 2-6 hours to complete the formation of the compound of formula VII

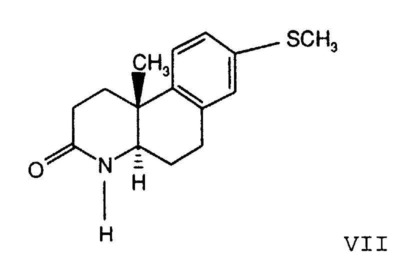

### Purification

The residue containing the product of formula VII may be dissolved, preferably in a haloalkane such as dichloromethane, washed with base, such as aqueous sodium bicarbonate, and concentrated under vacuum. Purification is afforded by recrystallization from e.g. ethyl acetate and methyl t-butyl ether or acetone or the like.

### N-Alkylation Process

It is necessary in the synthesis to methylate the nitrogen at the 4 position on the benzo[f]quinoline ring. U.S. Patent 5,239,075 shows such alkylation by reaction with an alkyl iodide in the presence of a strong base such as sodium hydride. An additional alkylation is shown in EPO publication 0703221.

The N-methylation comprises reacting a compound of the formula VII with a methyl halide, for example, methyl iodide in a reaction mixture comprising an organic solvent, for example a solvent chosen from the group consisting of tetrahydrofuran, dimethoxyethane, diethoxyethane and methyl *t*-butyl ether, and a base, for example, potassium *t*-butoxide and tetrahydrofuran, aqueous sodium or potassium hydroxide, to afford a compound of the formula VIII

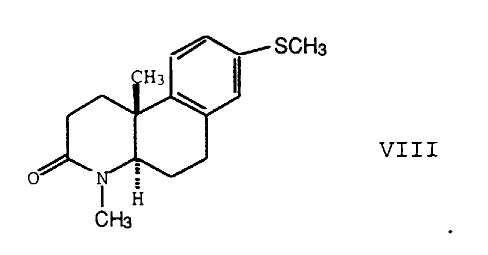

This alkylation process allows for an effective alkylation, under mild and easily controlled conditions, and allows for easy isolation of the products.

The alkylation process is carried out in conventional chemical plant equipment, preferably at ambient pressure and at moderate temperatures. It is preferably begun by slurrying the starting material of formula VII in the organic solvent at a temperature near ambient, such as from about 0° to about 50°, more preferably from about 15° to about 25°. The most preferred organic solvent is tetrahydrofuran, and it is preferred to use about 5-15 liters of solvent per kilogram of starting material; more preferable solvent volume is about 10 liters per kilogram. The alkyl iodide is then added as neat liquid. A substantial excess of alkyl iodide is preferably used, such as about 1.2-1.8 equivalents based on the starting material, most preferably about 1.5 equivalents.

The aqueous sodium or potassium hydroxide is then added, still at about ambient temperature, in an amount of about 1-4 liters per kilogram of starting material. The quantity of aqueous base is somewhat dependent on the concentration of the base and the choice of sodium or potassium hydroxide; when the most preferred base, 50% sodium hydroxide, is used, the most preferred amount of it is about 2 liters per kilogram of starting material. Then the reaction mixture, consisting of solid material slurried in two liquid phases, is warmed to about 25-65° with vigorous agitation and the reaction is allowed to proceed at about constant temperature with constant agitation. The preferred reaction temperature is about 35-40°. As the reaction proceeds toward completion, the solid starting material and alkyl iodide will dissolve and react, so the disappearance of solids is a crude indication of completion. The reaction may be followed by high pressure liquid chromatography on C-18 silica gel column, eluting with 1:1 acetonitrile:aqueous buffer (5% ammonium acetate) and monitoring at 220 nanometers.

When the reaction has gone as far as is desired toward completion, the mixture is cooled to about ambient and the aqueous layer is separated and discarded.

A preferred purification and isolation procedure proceeds by diluting the organic layer with water, and neutralizing it with aqueous mineral acid. Then the solution is distilled until the vapor temperature rises to about 69-80°, removing most of the tetrahydrofuran. Slow cooling to about 5° over a period of about 1-14 hours crystallizes the product, which needs only washing with water and drying to be ready for use as an intermediate or as a pharmaceutical. The most preferred isolation and purification is recrystallization of the residue after removal of tetrahydrofuran with ethyl acetate.

The alkylation process provides product in the same stereochemical form as the starting material, in satisfactory purity for the pharmaceutical industry, and in yields of or above 90% when operated according to the preferred manners.

### Electrophilic Coupling

The electrophilic coupling of the R¹ substituent to the sulfur group on the benzo[f]quinolinone ring may be afforded according to the following scheme V. 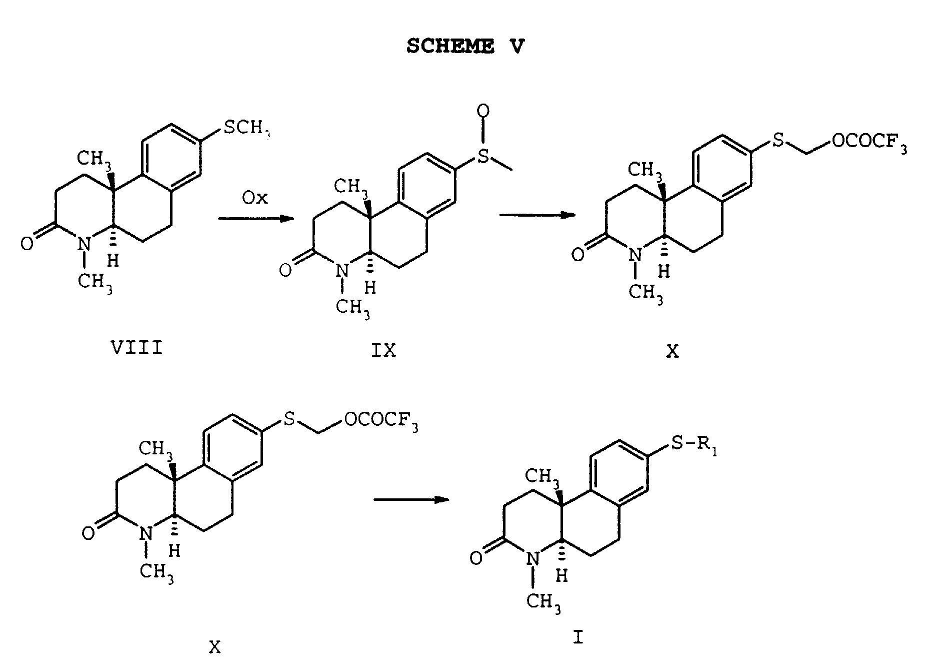 wherein R¹ is as defined above in formula I.
(+)-(4a*R*)-(10b*R*)-4-Methyl-8-methylthio-10b-methyl-1,2,3,4,4a,5,6,10b-octahydrobenzo[f]quinolin-3-one is oxidized to a sulfoxide compound. *m*-Chloroperoxybenzoic acid is a preferred oxidizing agent. With or without isolation, the sulfoxide compound is subjected to a Pummerer reaction as taught by Young, *et al., Tetrahedron Lett. 25,* 1753 (1984), such that the sulfoxide is reacted with an acylating agent, such as, trifluoroacetic anhydride to afford a trifluoroacetyloxymethylene sulfide compound (X). The trifluoroacetyloxymethylene sulfide compound is reacted with an electrophilic reagent, a hydride reducing reagent, such as sodium borohydride, potassium borohydride, lithium borohydride or the like, and a base, preferably a hydroxide or carbonate, most preferably, potassium carbonate, to prepare a compound of formula I. For the purpose of this reaction, suitable acylating agents include acyl halides, such as, acetyl chloride, sulfonic acid halides, reactive anhydrides, such as, trichloroacetic anhydride, phosphoric acid anhydride, sulfonic acid anhydride and like agents capable of yielding a Pummerer rearrangement product.

By methods known in the art, the electrophilic reagent is substituted with a leaving group, such as a halogen, sulfate, sulfonate or the like. The electrophilic reagent is then coupled to the sulfur on the benzo[f]quinolinone ring. A preferred electrophilic reagent is 2-chloro-4-ethylbenzothiazole and this reagent is coupled with the compound of formula X to afford (+)-(4a*R*)-(10b*R*)-4-methyl-8-(4-ethyl-2-benzothiazolylthio)-10b-methyl-1,2,3,4,4a, 5,6,10b-octahydrobenzo[f]quinolin-3-one.

Preferably a catalyst, for example tetrabutylammonium hydrogen sulfate is employed to direct the coupling of the trifluoroacetyloxymethylene sulfide with the electrophilic reagent. Sodium borohydride has been found to induce the reduction of the trifluoroacetyloxymethylene sulfide compound. Also, formaldehyde is generated *in situ* which is environmentally and pharmaceutically unacceptable. The present process reduces the formaldehyde as it is formed to methanol. Also, according to the present process, disulfides forming from air oxidation are reduced *in situ.* This process step allows starting materials to be more fully utilized and rigorous exclusion of oxygen, or impurities which promote oxidation, is unnecessary. Adding base to the mixture is not essential to promote the coupling. However, the decomposition of the borohydride is slowed, and the relative coupling rate is accelerated, with added base.

The following Preparations further illustrate the present inventive process. The Preparations are not intended to be limiting to the scope of the invention in any respect and should not be so construed.

Unless otherwise noted, starting materials were obtained from commercial suppliers and used without further purification. Toluene, dimethylformamide, and methylene chloride were stored over 4Å molecular sieves. Reactions using organometallic reagents were run under nitrogen. Reactions were monitored by high pressure liquid chromatography using the conditions specified below. Thin layer chromatography was done using Merck plates of Silica Gel 60 with a fluorescent indicator (F₂₅₄). ¹H and ¹³C NMR spectra were recorded on a General Electric QE or Bruker 300 MHz spectrophotometer at ambient temperature using CDCl₃ as solvent unless specified otherwise. NMR chemical shifts were recorded in ppm with solvent as the internal standard on the d scale and *J* values are in Hertz. IR, UV, and Mass Spec (MS) analyses were done by Eli Lilly Physical Chemistry Laboratory. High pressure liquid chromatography conditions: Hitachi model L-6200A Intelligent Pump with D-2500 chromato-integrator. 25 cm Zorbax RX C-18 column, 60:40 CH₃CN/H₂O, 1.0 mL/minute, 275 nm, Injection - 10 uL. Gas chromatography (GC) Conditions: HP 5890A GC with DB1 0.25 µ x 25 m column; 300 °C injection temperature; 300 °C detection (FID); column at 5 °C (5 minutes), 18 mL/minute to 250 °C then 250 °C for 20 minutes. The terms "NMR", "MS", "IR" and/or "GC" indicate that the product spectrum was analyzed and was consistent with the desired structure.

### Preparation 1

6-bromo-2-methyl ethers (A and B) 6-methylthio-2-methyl ethers (C and D) and 6-methylthio-2-tetralone(11). Trimethylorthoformate (26.7 mL, 0.24 mole) and thionyl chloride (260 mg, 2.2 mmole) were added to a slurry of 6-bromo-2-tetralone (50 g, 0.22 mole) in methanol (500 mL) under a nitrogen atmosphere. After stirring 3-5 hours at room temperature, the solvent was removed under reduced pressure. Heptane (500 mL) was added to the residue and the solvent was removed again under reduced pressure to yield a residual oil.
The residual oil was dissolved in tetrahydrofuran (400 mL) and cooled to -78°C under a nitrogen atmosphere. *n*-Butyl lithium (1.3 M in hexanes, 190 mL, 0.24 mole) was added to the solution followed 15 minutes later by the slow addition of methyl disulfide (23.8 mL, 0.26 mole). The mixture was stirred 10 minutes at -78°C and then allowed to warm to 10°C over 45 minutes. After the addition of 1N hydrochloric acid (200 mL) the mixture was concentrated under reduced pressure on a rotary evaporator (bath temperature 45°C) for 1 hour. Additional tetrahydrofuran (50 mL) was added to the mixture and the concentration was continued until hydrolysis of the enol ether intermediate was complete according to GLC. The mixture was then extracted with ethyl acetate (2 X 100 mL). The ethyl acetate layers were dried over Na₂SO₄ and concentrated under reduced pressure to a volume of 50 mL. Heptane (400 mL) was slowly added to the ethyl acetate concentrate and the resulting crystals were stirred 1 hour at 0°C, filtered, and dried to afford 30.6 g of 6-methylthio-2-tetralone (72% yield). mp 57-58°C GLC 99.5%

### Preparation 2a

### (+)-(4aR)-(10bR)-8-methylthio-10b-methyl-1,2,3,4,4a,5,6,10b-octahydrobenzo[f]quinolin-3-one.

A solution of 6-methylthio-2-tetralone (1 grams, 5.2 mmol, 1 equivalent) in dry toluene (18 mL) was treated with (R)-(+)-phenethylamine (0.72 ml, 5.7 mmol, 1.1 equivalent) and *p*-TsOH (6 mg). The solution was degassed 3 times with light vacuum/nitrogen and a positive nitrogen pressure was maintained. The solution was refluxed under Dean-Stark conditions to remove water. The progress of the imine formation was monitored by NMR. After 2.5 hours of refluxing, no starting ketone could be detected by ¹H NMR. Toluene was distilled off with light vacuum and under nitrogen, being careful not to expose the mixture to air. Dry tetrahydrofuran (14 mL) was added to obtain a light purple solution which was kept at -70°C under nitrogen. Lithium diisopropylamide was generated by dropwise addition of 2.5M hexanes solution of *n*-butyllithium (2.4 mL, 6.0 mmol, 1.15 equivalent) to a solution of diisopropylamine (0.78 mL, 6.0 mmol, 1.15 equivalent) in tetrahydrofuran (19 mL) at -45°C under nitrogen. The temperature was kept between -45°C and -30°C during the addition. After the addition, the solution was stirred for 10 minutes at -45°C. Upon cooling the lithium diisopropylamide solution to -75 C, the imine solution was added dropwise over 15 minutes via cannula while maintaining the temperature between -70 C and -75°C. The resulting yellowish-orange solution was allowed to warm to -20°C over 20 minutes and then recooled to -75°C. Iodomethane (0.36 ml, 5.8 mmol, 1.15 equivalents) was added between -75°C and -72°C. The solution was warmed (with the aid of an acetone bath) over 15 minutes to 0°C. Upon recooling to -5°C, methanesulfonic acid (0.43 mL, 6.6 mmol, 1.3 equivalents) was added over 2 minutes between -5°C and 1°C. After 5 minutes at 0°C, a gray heterogeneous mixture resulted. The mixture was recooled to -75°C. A 1.125M tetrahydrofuran solution of acrylic anhydride (11 mL, 12.5 mmol, 2.4 equivalents) was added quickly. The mixture was kept in the cooling bath for 15 hours, during which time it warmed to 13°C. The reaction was allowed to warm to 15°C. Water (2 mL) was added and the mixture stirred while warming the mixture to room temperature. The solution was diluted with diethyl ether (50 mL) and washed successively with 1N sodium hydroxide (20 mL), 1N hydrochloric acid (20 mL), water (20 mL), saturated aqueous sodium bicarbonate (40 mL), and brine (20 mL). The sodium sulfate solution was dried, concentrated and was flash chromatographed on silica gel (120 grams), eluting with 70:30 hexanes/ethyl acetate) to obtain 1.3 grams (69% yield) of (+)-(10b*R*)-4-(2-(R)-phenethyl)-8-methylthio-10b-methyl-1,2,3,4,6,10b-hexahydrobenzo[f]quinolin-3-one (NMR). A mixture of triethylsilane (12 mL, 75 mmol) and trifluoroacetic acid (14.5 mL, 188 mmol) precooled to -15°C under nitrogen was added to (+)-(10b*R*)-4-(2-(R)-phenethyl)-8-methylthio-10b-methyl-1,2,3,4,6,10b-hexahydrobenzo[f]quinolin-3-one (2.76 grams, 7.6 mmol) precooled in -15°C bath. The mixture was stirred for 15 hours, during which time it warmed to 13°C. Thin layer chromatography (70:30 hexanes/ethyl acetate) and high pressure liquid chromatography showed complete disappearance of (+)-(10b*R*)-4-(2-(R)-phenethyl)-8-methylthio-10b-methyl-1,2,3,4,6,10b-hexahydrobenzo[f]-quinolin-3-one and the appearance of a new product, indicating complete double bond reduction. The mixture was then refluxed for 2 hours to remove the chiral auxiliary. Upon cooling to room temperature, the mixture was concentrated at reduced pressure. The residue was taken up in methylene chloride (50 mL) and washed twice with saturated aqueous sodium bicarbonate (35 mL) and brine (50 mL). The dried sodium sulfate and concentrated crude product was purified by flash chromatography on silica gel (100 grams), eluting first with 3:1 hexanes/ethyl acetate to remove triethylsilane and then with 14% methanol in methylene chloride with 1% acetic acid to obtain a foamy solid. Recrystallization from hot ethyl acetate gave 1.6 grams (82%) of (+)-(4a*R*)-(10b*R*)-8-methylthio-10b-methyl-1,2,3,4,4a,5,6,10b-octahydrobenzo[f]quinolin-3-one (92%ee, IR, NMR, MS). Calculated for C₁₅H₁₉NOS: C, 68.93; H, 7.33. Found C, 69.05; H, 7.44.

### Preparation 2b

### (+)-(4aR)-(10bR)-8-methylthio-10b-methyl-1, 2, 3, 4, 4a, 5, 6, 10b-octahydrobenzo[f}quinolin-3-one.

A solution of compound 6-methylthio-2-tetralone (9.6 g, 50 mmol), *p*-toluenesulfonic acid monohydrate (40 mg, 0.2 mmol) and *R*-(+)-a-methylbenzylamine (6.64 g, 55 mmol) in toluene (150 mL) was heated to reflux. Water generated during the reaction was removed via a Dean -Stark trap. The progress of the reaction was monitored by NMR (300 mHz, CDCl₃) which indicated 100% conversion after 2 hours of refluxing. The enamine solution was concentrated to a volume of 30 mL by atmospheric distillation. The enamine solution was then cooled to -78°C, diluted with tetrahydrofuran (100 mL) and added via cannula to a solution of lithium diisopropyl amide (2.0 M in heptane/THF) (28.7 mL, 57.5 mmol) and tetrahydrofuran (130 mL). After stirring for 30 minutes at -78 °C, iodomethane (3.6 mL, 57.5 mmol) was introduced and stirring was continued for another 30 minutes at -78 °C. Methanesulfonic acid (4.05 mL, 62.5 mmol) was added followed by warming of the reaction mixture to 0 °C. In a separate vessel, acrylic acid (6.6 g, 91.5 mmol) was slowly added to a solution of triethylamine (9.25 g, 91.5 mmol), hydroquinone (0.11 g) and 2,6-di-tert-butyl-4-methylphenol (0.11 g) in tetrahydrofuran (100 mL) at -10 °C to -15 °C. Trimethylacetyl chloride (11.0 g, 91.5 mmol) was slowly added, keeping the temperature less than -5 °C. The reaction mixture was stirred 2 hours at -10 °C, then filtered and the filtered solids washed with 20 mL of tetrahydrofuran. The filtrate and sodium bicarbonate (NaHCO₃) (9.2 g, 110 mmol) were added to the enamine solution and the resulting mixture was stirred at 0°C for 4 hours. Deionized water (90 mL) was added and stirring continued for 0.5 hours at 0°C. The solvent was then removed by vacuum distillation (temperature <30°C). Methylene chloride (90 mL) was added and the layers separated. The methylene chloride layer was washed sequentially witha 1N NaOH solution (4 X 25 mL), a 1 N hydrochloric acid solution (20 mL) and a saturated sodium bicarbonate solution (20 mL). The solvent was then removed from the reaction mixture by vacuum distillation (temperature <30°C). Triethylsilane (80 mL, 500 mmol) and trifluoroacetic acid (100 mL, 1.3 mol) were added and the resulting mixture was stirred at 27-32°C for 12 hours followed by 6 hours at reflux. Progress of the reaction was monitored by HPLC (such as 25 cm Supelcosil LC-ABZ, isocratic 40% deionized water / 60% acetonitrile, 220 nm, 2 mL/min) which indicated >95% conversion. The reaction mixture was then concentrated by vacuum distillation to approximately 70 mL and diluted by the addition of acetonitrile (70 mL). The acetonitrile solution was washed with heptane (6 X 25 mL). The acetonitrile was removed by vacuum distillation and the residue dissolved in methylene chloride (50 mL). A saturated solution of sodium bicarbonate (150 mL) was slowly added and the layers were separated. The methylene chloride layer was washed with a saturated solution of sodium bicarbonate (150 mL) and the solvent removed by vacuum distillation. The residue was diluted with ethyl acetate (40 mL) and the resulting mixture was stirred at -30°C for 1 hour. The mixture was filtered and the filter cake washed with cold (-30 °C) ethyl acetate (10 mL) followed by drying at 50°C to give 6.22 g (49%) of (+) - (4a*R*) - (10b*R*) -8-methylthio-10b-methyl-1, 2, 3, 4, 4a, 5, 6, 10b-octahydrobenzo[f}quinolin-3-one (GLC purity 99.5%).

### Preparation 3

### (+)-(4aR)-(10bR)-4-methyl-8-methylthio-10b-methyl-1,2,3,4,4a,5,6,10b-octahydrobenzo[f]quinolin-3-one.

(+)-(4a*R*)-(10b*R*)-8-methylthio-10b-methyl-1,2,3,4,4a,5,6,10b-octahydrobenzo[f]quinolin-3-one (0.423 grams, 1.62 mmol, 1.0 equivalents) was treated with potassium t-butoxide (1.86 mL as a 1.0 M solution in tetrahydrofuran, 1.15 equivalents) in dimethylformamide (1.6 mL) at approximately 0°C. After stirring 5 minutes, methyl iodide (0.116 mL, 1.15 equivalents) was added and the mixture allowed to stir for 2 hours at 0°C. The mixture was then treated with approximately 100 mg of acetic acid and solvents were removed in a stream of nitrogen. The solid was dissolved in approximately 50 mL of methylene chloride and washed twice with water. The extracts were dried (4 molecular sieves) and filtered over silica gel (2 grams, washed with ethyl acetate containing 2% methanol). The solid from the evaporation of the methylene chloride extracts was chromatographed on silica gel with methylene chloride, ethyl acetate and methanol (50:50:1) as the eluent. (GC).

### Preparation 4

### (+)-(4aR)-(10bR)-4-methyl-8-(4-ethyl-2-benzothiazolylthio)-10b-methyl-1,2,3,4,4a,5,6,10b-octahydrobenzo[f]quinolin-3-one.

A solution of meta-chloroperoxybenzoic acid (approximately 56% potency, 6.32 grams as a solution in methylene chloride) was added to a mixture of (+)-(4a*R*)-(10b*R*)-4-methyl-8-methylthio-10b-methyl-1,2,3,4,4a,5,6,10b-octahydrobenzo[f]-quinolin-3-one.(5.50 grams in 110 mL of methylene chloride) and aqueous sodium bicarbonate (9.4 grams in 110 mL of water) at 0 to -1°C over approximately one hour. After 8 hours, the layers were separated and the organic extracts were washed twice with aqueous 1% sodium bicarbonate, dried (4Å molecular sieves) and evaporated to a total weight of 5.90 grams. A portion (2.91 grams) of this non-purified sulfoxide in 10 mL of toluene-*d*_{*8*} was treated with 2.20 mL of trifluoroacetic anhydride at 5-10°C. After 30 minutes., ¹H NMR analysis of a 0.50 mL aliquot showed none of the starting sulfoxide (ArSOCH₃ singlet absent at d = 2.25 ppm) and the exclusive formation of the Pummerer product (ArSCH₂OCOCF₃, singlet at d = 5.31 ppm). The reaction mixture was subjected to vacuum (5-10 torr) for 30 minutes after which the contents were added to a stirred mixture (under nitrogen) of water (20 mL), tetra-*n*-butylammonium hydrogen sulfate (0.1 gram), and 7.1 mL of water soluble borohydride (12% by weight of sodium borohydride in 14 N sodium hydroxide) at 5-10°C over 20 minutes. After an additional 20 minutes, 2-chloro-4-ethylbenzothiazole (3.00 grams with an additional 9.5 mL of toluene-*d*₈) and another portion of tetra-*n*-butylammonium hydrogen sulfate (0.50 gram) were added. The reaction was stirred at 37-39°C during which, four 0.25-mL portions were removed for direct ¹H NMR assay and high pressure liquid chromatography for analysis of the conversion and product distribution. After 26 hours the top-most layer (toluene containing (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one and excess 2-chloro-4-ethylbenzothiazole) of the three phase mixture was separated and diluted with 25 mL of methylene chloride. This organic layer was washed with aqueous 1 N sulfuric acid, aqueous 5% sodium bicarbonate, dried with 4 Å molecular sieves, and concentrated under vacuum to 5.24 grams. The resulting solid was digested with hot methyl *t*-butyl ether (30 mL) after which it was concentrated under vacuum to 20 mL and then cooled to 0°C. The mixture was filtered and the white solid was dried affording 2.92 grams of (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one (IR, NMR, MS). Calculated for C₂₄H₂₆N₂OS₂: C, 67.93; H, 6.16; N, 6.83; S, 15.08. Found C, 68.21; H, 6.20; N, 6.63; S, 15.17.

### Preparation 5

### (+)-(4aR)-(10bR)-4-methyl-8-(4-ethyl-2-benzothiazolylthio)-10b-methyl-1,2,3,4,4a,5,6,10b-octahydrobenzo[f]quinolin-3-one.

A solution of *meta*-chloroperoxybenzoic acid (35.2 mmole in 100 mL of methylene chloride) was added to (+)-(4a*R*)-(10b*R*)-methyl-8-methylthio-10b-methyl-1,2,3,4,4a,5,6,10b-octahydrobenzo[f]quinolin-3-one (10.0 grams, 98% purity by weighted standard potency assay, 35.6 mmoles) in a biphasic mixture of methylene chloride (200 mL) and aqueous sodium bicarbonate (8.90 grams in 89 mL water) at -2 to 0°C over a period of 1 hour. The layers were separated and the methylene chloride layers washed once with sodium metabisulfite (1.00 gram in 25 mL) and three times with sodium bicarbonate (1.00 gram in 100 mL). The dried (4 Å molecular sieves) methylene chloride extract was concentrated to approximately 20 mL and diluted with 100 mL of toluene. The mixture was then concentrated under vacuum at 30-35°C. This process was repeated twice with fresh toluene (100 mL portions each) after which the sulfoxide (usually crystalline) was diluted with toluene (100 mL). The mixture was then treated with trifluoroacetic anhydride (7.30 mL) over 10 minutes at 0 to 10°C. After 30 minutes at 0°C, this solution was subjected to vacuum (<10 torr) for 30 minutes and added over 30 minutes to a degassed (nitrogen) mixture of potassium carbonate (41 grams), sodium borohydride (2.88 grams), tetra-*n*-butylammonium hydrogen sulfate (2.00 grams), and 2-chloro-4-ethylbenzothiazole (8.20 grams at 96.5% purity dissolved in 5 mL of toluene) and water (87 mL) at 0-5°C. This mixture was warmed to 40°C over one hour and then stirred at 40°C for 26 hours. The toluene layer was separated (while warm) and washed with 3x100-mL portions of water. The toluene layer was diluted with 125 mL ethyl acetate and then washed in sequence with 3x200 mL portions of 0.25 N hydrochloric acid, 100 mL of 1% sodium bicarbonate and the 100 mL of saturated aqueous sodium chloride. The organic layer was dried (10 grams of 4Å molecular sieves), concentrated to a total volume of 25 mL and treated with 100 mL of methyl *t*-butyl ether (refluxed for 30 minutes then, 0°C for 1 hour) affording (+)-(4a*R*)-(10b*R*)-4-methyl-8-(4-ethyl-2-benzothiazolylthio)-10b-methyl-1,2,3,4,4a,5,6,10b-octahydrobenzo [f]quinolin-3-one.

### Preparation 6

### (+)-(4aR)-(10bR)-4-methyl-8-(4-ethyl-2-benzothiazolylthio)-10b-methyl-1,2,3,4,4a,5,6,10b-octahydrobenzo[f]quinolin-3-one.

The dry sulfoxide IX (96% purity by gas chromatography, 8.90 grams, 29.4 mmol corrected) as a suspension in toluene (89 mL) was treated dropwise with trifluoroacetic anhydride (5.2 mL) at 0-5°C in 20 minutes (dissolution occurs). After 30 minutes, the solution was added to a mixture of potassium carbonate (32 grams), water (45 mL), tetrabutylammonium hydrogen sulfate (2.25 grams), sodium borohydride (1.0 grams), the 2-chloro-4-ethylbenzothiazole (7.07 grams, approximately 96.5 % purity by gas chromatography), and toluene (10 mL) at approximately 5-15°C over 30 minutes. The biphasic mixture was then stirred for 20 hours at 43°C after which a small amount of solid was filtered from the reaction mixture. The warm toluene layer was washed once with 400 mL of water (45°C) and then evaporated under vacuum yielding 13.86 grams of white solid consisting of mainly (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one and 2-chloro-4-ethylbenzothiazole. 13.0 grams of this mixture was treated with methyl *t*-butyl ether (50 mL at reflux then 0°C for 2 hours) affording 11.05 grams of (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one (IR, NMR, MS). Calculated for C, 68.21; H, 6.20; N, 6.63. Found C, 68.29; H, 6.15; N, 6.67.

### Preparation 7

(+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one. *meta*-Chloroperoxybenzoic acid (approximately 92 grams, approximately 50% potency, in 1.0 L of methylene chloride) was added at 0°C to a solution of (+)-(4a*R*)-(10b*R*)-4-methyl-8-methylthio-10b-methyl-1,2,3,4,4a,5,6,10b-octahydrobenzo[f] quinolin-3-one (79.5 grams, 92.9% potency, 0.269 moles) in methylene chloride (2.2 L). The progress of the reaction was monitored by high pressure liquid chromatography (240 nm) for the oxidation of (+)-(4a*R*)-(10b*R*)-4-methyl-8-methylthio-10b-methyl-1,2,3,4,4a,5,6,10b-octahydrobenzo[f]-quinolin-3-one to a level of less than 0.3% by area. The organic solution was stirred with a sodium bisulfite solution (75 grams in 1 L deionized water). The organic layer was separated, and washed with 6% sodium bicarbonate solution (3 X 1 L). The organic layer was dried over sodium sulfate and concentrated. Toluene (1 L) was added to the intermediate sulfoxide and this solution was concentrated under vacuum. This was repeated twice with fresh toluene (1.1 L each) after which the sulfoxide was dissolved in 1.1 L of toluene and cooled in an ice bath. Trifluoroacetic anhydride (51 mL) was added dropwise to the sulfoxide at 0°C over 15 minutes. After 30 minutes of stirring at 0°C, the Pummerer product (ArSCH₂OCOCF₃) was added via cannula to a well stirred mixture of deionized water (414 mL), potassium carbonate (319 grams), sodium borohydride (15.2 grams), 2-chloro-4-ethylbenzothiazole (65.7 grams approximately 96.5% purity), tetrabutylammonium hydrogen sulfate (21.6 grams), and toluene (170 mL) at 10°C. The reaction was heated slowly to 42°C while monitoring the progress by high pressure liquid chromatography. After 18 hours, an additional volume of toluene (1.0 L) was added and the toluene layers washed with deionized water (3x1 L, 45°C). To the organic layer was added ethyl acetate (1 L) then the organic layer washed with 0.25 M hydrochloric acid solution (3x1 L), 1N sulfuric acid solution (3x1 L), 6% sodium bicarbonate solution (1.5 L), and saturated sodium chloride solution (2 L). The organic extracts were dried over 4Å molecular sieves (500 grams) then concentrated. Methyl *t*-butyl ether was added (400 mL) and the mixture heated to reflux. After 30 minutes of stirring at reflux the mixture was cooled to 5°C. The suspension was filtered and washed with methyl *t*-butyl ether (100 mL). The solution was filtered and the product was dried at 50°C at approximately 5 mm for 18 hours affording 90.1 grams (80%) of (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one.

## Claims

1. A process for preparing a compound of the formula I 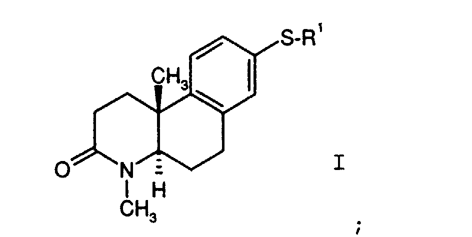 wherein R¹ represents:
2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted with 1-3 groups chosen from the group consisting of trifluoromethyl, trifluoroethoxy, C₁-C₄ alkyl, trifluoromethoxy, hydroxy, C₁-C₃ alkoxy, nitro, C₁-C₃ alkylthio, C₁-C₆ alkanoyl, phenyl, oxo, phenoxy, phenylthio, C₁-C₃ alkylsulfinyl, C₁-C₃ alkylsulfonyl, cyano, amino, C₁-C₃ alkylamino, diphenylmethylamino, triphenylmethylamino, benzyloxy, benzylthio, (mono-halo, nitro or CF₃)benzyl(oxy or thio), dil(C₁-C₃ alkyl, C₃-C₆ cycloalkyl, or C₄-C₈ cycloalkylalkyl)amino, (mono-C₁-C₃ alkyl, C₁-C₃ alkoxy or halo)(phenyl, phenoxy, phenylthio, phenylsulfonyl or phenoxysulfonyl), C₂-C₆ alkanoylamino, benzoylamino, diphenylmethylamino(C₁-C₃ alkyl), aminocarbonyl, C₁-C₃ alkylaminocarbonyl, di(C₁-C₃ alkyl)aminocarbonyl, halo-C₁-C₆ alkanoyl, aminosulfonyl, C₁-C₃ alkylaminosulfonyl, di(C₁-C₃ alkyl)aminosulfonyl, phenyl(oxy or thio)(C₁-C₃ alkyl), (halo, C₁-C₃ alkyl or C₁-C₃ alkoxy)phenyl(oxy or thio)(C₁-C₃ alkyl), benzoyl, or (amino, C₁-C₃ alkylamino or di(C₁-C₃ alkyl)amino)(C₁-C₃ alkyl);
which comprises: converting a ketone of the formula 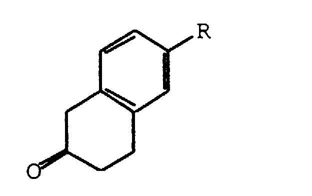 wherein R is halogen;
to a protected ether;
reacting the protected ether with a reactive alkyllithium compound and a sulfur transfer reagent to afford an S-methylated ether compound;
deprotecting the S-methylated ether compound to afford a methylthiotetralone compound of the formula II 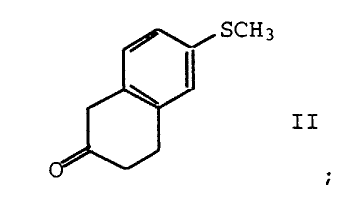 reacting the compound of formula II with (*R*)-(+)-phenethylamine to afford a compound of the formula III 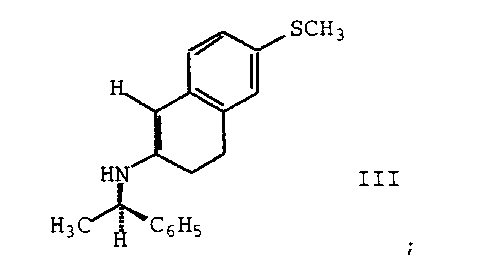 reacting the compound of formula III with a strong lithium base to afford a lithioenamine compound of the formula IV 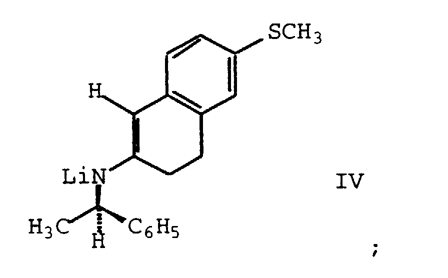 methylating the resulting lithioenamine of the formula IV to a compound of the formula V 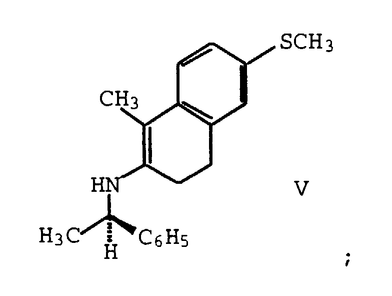 reacting the compound of formula V with an acyl halide or an anhydride of acrylic acid to prepare a compound of the formula VI 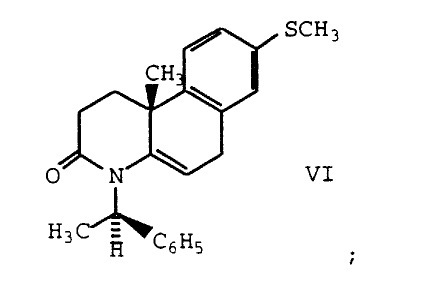 quenching the reaction with base, and combining the residue comprising the compound of formula VI with an appropriate silane and trifluoroacetic acid in the absence of a solvent to prepare a compound of the formula VII 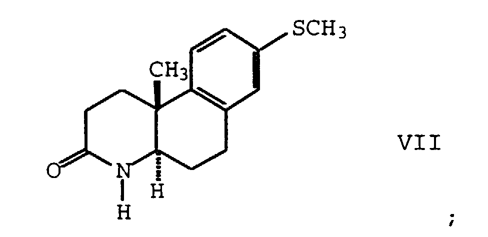 reacting the compound of formula VII with a methyl halide in a reaction mixture comprising an organic solvent and a strong base to afford an arylmethylsulfide compound of the formula VIII 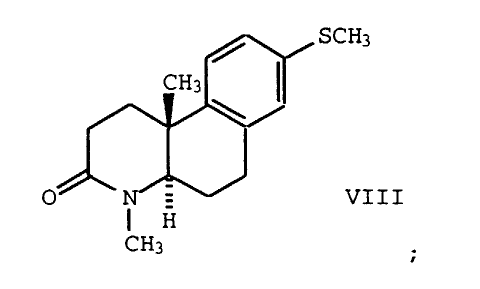 oxidizing the compound of formula VIII to a sulfoxide compound of the formula IX 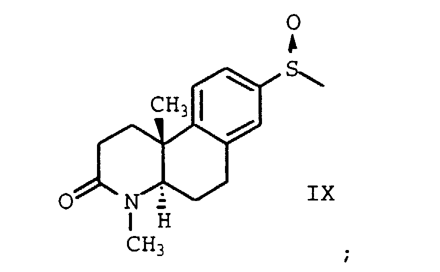 reacting the sulfoxide compound of the formula IX with an acylating agent to afford a Pummerer rearrangement product;
reacting the Pummerer rearrangement product with an electrophile selected from the group consisting of A-R¹ wherein A is a leaving group, in the presence of a phase transfer catalyst, a hydride reducing reagent and a base, to prepare a compound of the formula I.

2. A process for preparing the compound of formula II 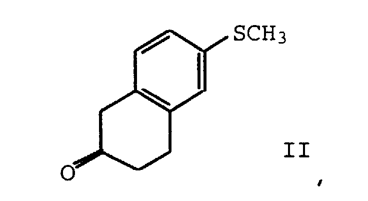 which comprises: converting a ketone of the formula 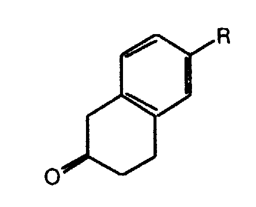 wherein R is halogen;
to a protected ether;
reacting the protected ether with a reactive alkyllithium compound and a sulfur transfer reagent to afford an S-methylated ether compound;
deprotecting the S-methylated ether compound to afford the methylthiotetralone compound of the formula II.

3. The process of Claim 2, wherein R is bromo, the sulfur transfer reagent is dimethyl disulfide and trimethyl orthoformate is employed to produce the protected ethers A and B:

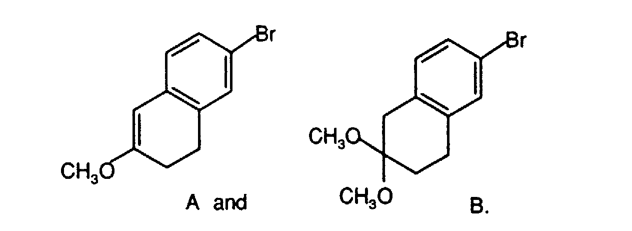

4. The process of Claim 3, wherein the S-methylated ether compounds are C and D:

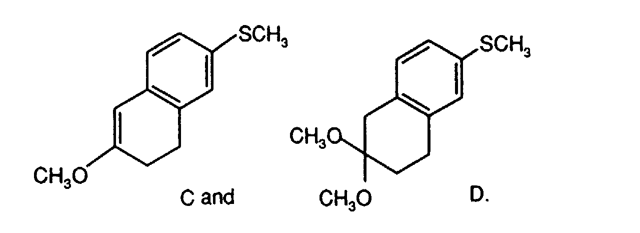

5. The process of Claim 2, wherein the halogen is bromo; the protected ether is prepared by employing trimethyl orthoformate in methanol in the presence of an acid catalyst; the reactive alkyllithium compound is *n*-butyllithium; the sulfur transfer reagent is dimethyl disulfide and the S-methylated ether compound is deprotected by employing hydrochloric acid.

6. The process of Claim 5, wherein the 6-bromo-2-tetralone is prepared by reacting 4-bromophenylacetic acid with methylene chloride and a reagent selected from thionyl chloride, phosphorus trichloride, oxalyl chloride, and phosphorus pentachloride followed by reaction with ethylene gas in the presence of a Lewis acid catalyst.

7. The process of any one of Claims 2, 3, 4, 5 or 6, further comprising converting the compound of formula II 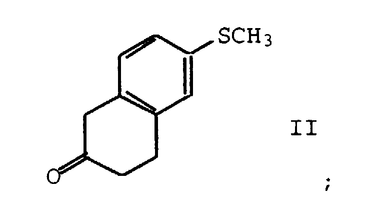 to a compound of the formula I 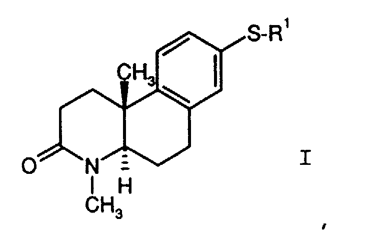 wherein R¹ represents:
2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted with 1-3 groups chosen from the group consisting of trifluoromethyl, trifluoroethoxy, C₁-C₄ alkyl, trifluoromethoxy, hydroxy, C₁-C₃ alkoxy, nitro, C₁-C₃ alkylthio, C₁-C₆ alkanoyl, phenyl, oxo, phenoxy, phenylthio, C₁-C₃ alkylsulfinyl, C₁-C₃ alkylsulfonyl, cyano, amino, C₁-C₃ alkylamino, diphenylmethylamino, triphenylmethylamino, benzyloxy, benzylthio, (mono-halo, nitro or CF₃)benzyl(oxy or thio), di(C₁-C₃ alkyl, C₃-C₆ cycloalkyl, or C₄-C₈ cycloalkylalkyl)amino, (mono-C₁-C₃ alkyl, C₁-C₃ alkoxy or halo)(phenyl, phenoxy, phenylthio, phenylsulfonyl or phenoxysulfonyl), C₂-C₆ alkanoylamino, benzoylamino, diphenylmethylamino(C₁-C₃ alkyl), aminocarbonyl, C₁-C₃ alkylaminocarbonyl, di(C₁-C₃ alkyl)aminocarbonyl, halo-C₁-C₆ alkanoyl, aminosulfonyl, C₁-C₃ alkylaminosulfonyl, di(C₁-C₃ alkyl)aminosulfonyl, phenyl(oxy or thio)(C₁-C₃ alkyl), (halo, C₁-C₃ alkyl or C₁-C₃ alkoxy)phenyl(oxy or thio)(C₁-C₃ alkyl), benzoyl, or (amino, C₁-C₃ alkylamino or di(C₁-C₃ alkyl)amino)(C₁-C₃ alkyl).

8. The process of any one of Claims 2, 3, 4, 5 or 6, further comprising converting the compound of the formula II 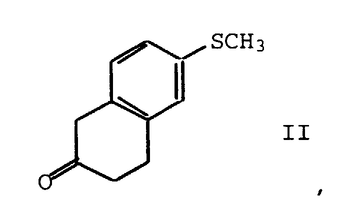 to (+)-(4a*R*)-(10b*R*)-4-methyl-8-(4-ethyl-2-benzothiazolylthio)-10b-methyl-1,2,3,4,4a,5,6,10b-octahydrobenzo[f]quinolin-3-one.

9. The process as in any one of Claims 2, 3, 4, 5, or 6, further comprising: reacting the compound of the formula II 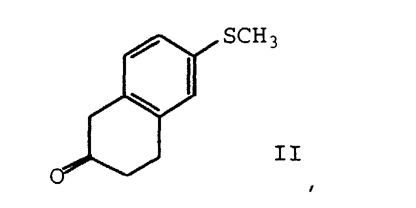 with (*R*)-(+)- phenethylamine to afford a compound of the formula III 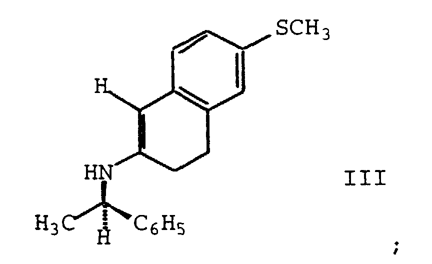 reacting the compound of formula III with a strong lithium base to afford a lithioenamine compound of the formula IV 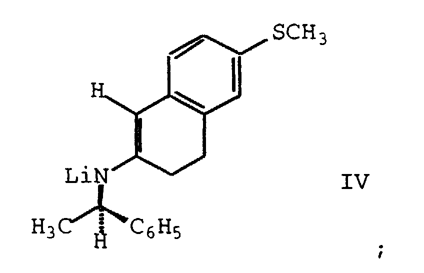 methylating the resulting lithioenamine of the formula IV to a compound of the formula V 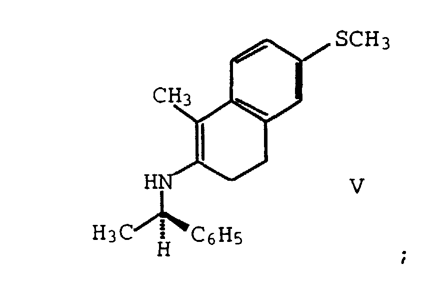 reacting the compound of formula V with an acyl halide or an anhydride of acrylic acid to prepare a compound of the formula VI 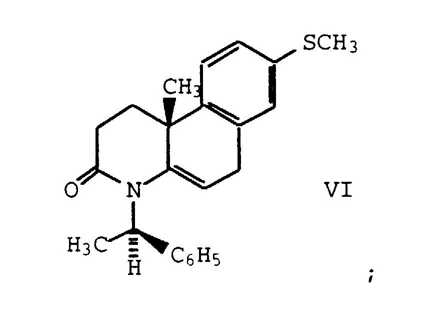 quenching the reaction with base, and combining the residue comprising the compound of formula VI with an appropriate silane and trifluoroacetic acid in the absence of a solvent to prepare a compound of the formula VII

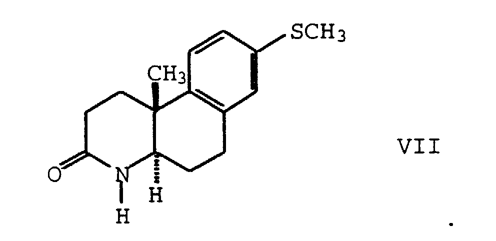

10. The process of Claim 9, further comprising: reacting the compound of formula VII with a methyl halide in a reaction mixture comprising an organic solvent and a strong base to afford an arylmethylsulfide compound of the formula VIII 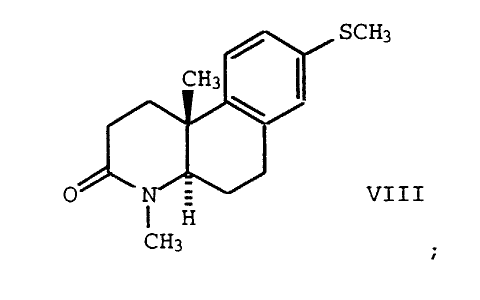 oxidizing the compound of formula VIII to a sulfoxide compound of the formula IX 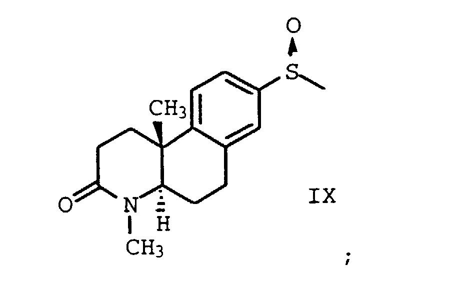 reacting the sulfoxide compound of the formula IX with an acylating agent to afford a Pummerer rearrangement product;
reacting the Pummerer rearrangement product with an electrophile selected from the group consisting of A-R¹ wherein A leaving group and R¹ represents:
2-nitrophenyl, 4-nitrophenyl, 2-cyanophenyl, 4-cyanophenyl, 2-nitronaphthyl, 4-nitronaphthyl, 2-cyanonaphthyl, 4-cyanonaphthyl, 2-quinolinyl, 4-quinolinyl, 7-quinolinyl, 1-isoquinolinyl, 3-isoquinolinyl, 8-isoquinolinyl, 2-quinoxalinyl, 2-benzothiazolyl, 3-1H-indazolyl, 2-benzoxazolyl, 3-1,2-benzisothiazolyl, 2-pyridinyl, 4-pyridinyl, 2-pyrazinyl, 2-naphtho[2,3-d]thiazolyl, 2-naphtho[1,2-d]thiazolyl, 9-anthryl, 2-thiazolyl, 2-benzimidazolyl, 1-benz[g]isoquinolinyl, 8-benz[g]isoquinolinyl, 5-1H-tetrazolyl, 2-quinazolinyl, 2-thiazolo[4,5-b]pyridinyl, 4-10H-pyridazino[3,2-b]-2-quinazolinyl, 2-1,4-benzodioxinyl, 2-triazine, 2-benzoxazine, 4-benzoxazine, 2-purine or 8-purine;
wherein the above R¹ groups are unsubstituted or substituted with 1-3 groups chosen from the group consisting of trifluoromethyl, trifluoroethoxy, C₁-C₄ alkyl, trifluoromethoxy, hydroxy, C₁-C₃ alkoxy, nitro, C₁-C₃ alkylthio, C₁-C₆ alkanoyl, phenyl, oxo, phenoxy, phenylthio, C₁-C₃ alkylsulfinyl, C₁-C₃ alkylsulfonyl, cyano, amino, C₁-C₃ alkylamino, diphenylmethylamino, triphenylmethylamino, benzyloxy, benzylthio, (mono-halo, nitro or CF₃)benzyl(oxy or thio), di(C₁-C₃ alkyl, C₃-C₆ cycloalkyl, or C₄-C₈ cycloalkylalkyl)amino, (mono-C₁-C₃ alkyl, C₁-C₃ alkoxy or halo)(phenyl, phenoxy, phenylthio, phenylsulfonyl or phenoxysulfonyl), C₂-C₆ alkanoylamino, benzoylamino, diphenylmethylamino(C₁-C₃ alkyl), aminocarbonyl, C₁-C₃ alkylaminocarbonyl, di(C₁-C₃ alkyl)aminocarbonyl, halo-C₁-C₆ alkanoyl, aminosulfonyl, C₁-C₃ alkylaminosulfonyl, di(C₁-C₃ alkyl)aminosulfonyl, phenyl(oxy or thio)(C₁-C₃ alkyl), (halo, C₁-C₃ alkyl or C₁-C₃ alkoxy)phenyl(oxy or thio)(C₁-C₃ alkyl), benzoyl, or (amino, C₁-C₃ alkylamino or di(C₁-C₃ alkyl)amino)(C₁-C₃ alkyl);
in the presence of a phase transfer catalyst, a hydride reducing reagent and a base, to prepare a compound of formula I

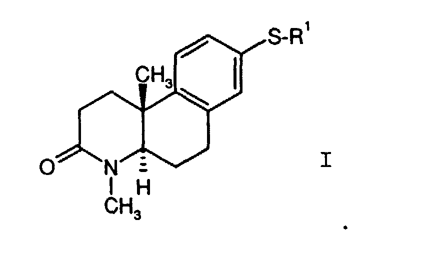

11. A compound of the formula A:

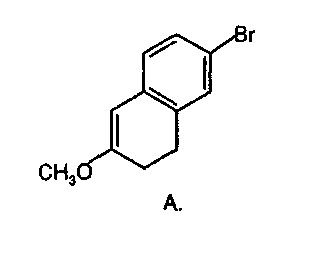

12. A compound of the formula B:

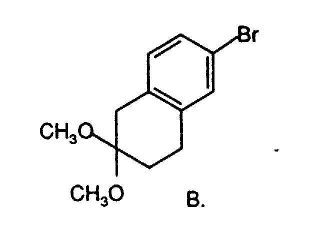

13. A compound of the formula C:

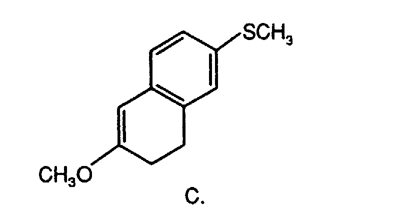

14. A compound of the formula D:
